# EUROPEAN PATENT SPECIFICATION

(11) **EP 3 313 408 B1**
(45) Date of publication and mention of the grant of the patent: **06.12.2023**
(21) Application number: 16731177.8
(22) Date of filing: 23.06.2016
(51) Int. Cl.: A61K 31/585, A61P 15/18

(54) **DROSPIRENONE-BASED CONTRACEPTIVE FOR A FEMALE PATIENT AFFECTED WITH EXCESS WEIGHT**
DROSPIRENONBASIERTES VERHÜTUNGSMITTEL FÜR EINEN WEIBLICHEN ÜBERGEWICHTIGEN PATIENTEN
CONTRACEPTIF À BASE DE DROSPIRENONE POUR UN PATIENT FÉMININ AYANT UN EXCÈS DE POIDS

(30) Priority: 23.06.2015 CN 201510348953; 23.06.2015 EP 15305965
(43) Date of publication of application: 02.05.2018
(73) Proprietor: Laboratorios Leon Farma SA, 24008 Leon (ES)
(72) Inventor: DROUIN, Dominique, 91370 Verrieres (FR); BOYER-JOUBERT, Cécile, 92260 Fontenay aux Roses (FR); PERRIN, Philippe, 75015 Paris (FR)
(74) Representative: Hoffmann Eitle
(86) International application number: PCT/EP2016/064574
(87) International publication number: WO 2016/207298

(56) References cited:
- WO-A1-2012/000981
- DINGER J C ET AL: "Oral contraceptive effectiveness according to body mass index, weight, age, and other factors", AMERICAN JOURNAL OF OBSTETRICS & GYNECOLOGY, MOSBY, ST LOUIS, MO, US, vol. 201, no. 3, 1 September 2009 (2009-09-01), pages 263.e1-263.e9, XP026557648, ISSN: 0002-9378, DOI: 10.1016/J.AJOG.2009.03.017 [retrieved on 2009-05-30]
- BLANCO-MOLINA M A ET AL: "Progestin-only contraception and venous thromboembolism", THROMBOSIS RESEARCH, TARRYTOWN, NY, US, vol. 129, no. 5, 22 February 2012 (2012-02-22), pages e257-e262, XP028419802, ISSN: 0049-3848, DOI: 10.1016/J.THROMRES.2012.02.042 [retrieved on 2012-02-29]

## Description

### FIELD OF THE INVENTION

The present disclosure relates to the field of contraceptive compositions and methods, in particular for a female patient affected with excess weight, which encompasses a female patient affected with overweight or obesity.

### BACKGROUND OF THE INVENTION

Excess weight among adults, i.e. overweight and obesity, highly increases with time in the general population.

The World Health Organization (WHO) has established weight standard status categories based on a body mass index (BMI), which range for adults are as follows:
- an underweight status is featured by a BMI below 18.5 kg/m²;
- a normal weight status is featured by a BMI ranging from 18.5 kg/m² to 24.9 kg/m²;
- an overweight status is featured by a BMI ranging from 25.0 kg/m² to 29.9 kg/m²; and
- an obesity status is featured by a BMI of 30.0 kg/m² or greater.

Based on the latest OECD Health Statistics report, the majority of adults (53%) are overweight or obese in EU countries with obese adults representing about 17% of the adult population (OECD/European Union (2014), "Overweight and obesity among adults", in Health at a Glance: Europe 2014, OECD Publishing.). In the United States, 64% of women are affected with overweight or obesity, among which 36% are obese (Flegal et al., Journal of the American Medical Association. 2012; 307(5):491-97). Obesity is a known risk factor for numerous health problems, including hypertension, high cholesterol, diabetes, cardiovascular diseases, and some forms of cancer.

The prevalence of unintended pregnancy rivals that of overweight or obesity. About 40% of all pregnancies worldwide were unintended, with 43% in Europe and 51% in North America (Sedgh G. et al. Studies in Family Planning 2014; 45 (3):301-314). Obese women and especially those with comorbidities are at higher risk of pregnancy-related complications. Thus, avoidance of unintended pregnancy in these women is especially important (Cedergren MI. Obstet. Gynecol. 2004;103:219-24).

The best way to prevent unintended pregnancies is to provide appropriate contraception with safe and well-tolerated options.

Hormonal contraceptives are one of the most widely used contraceptive methods in the world and have the widest geographic distribution of any method. Hormonal contraceptives are also the most popular form of reversible female contraception in the developed world (United Nations. Department of Economic and Social Affairs - Population Division. World Contraceptive Patterns 2013.). Among hormonal contraceptives, one may cite combined contraceptives (pills, vaginal rings or transdermal patches) or progestin-only contraceptives (progestin-only pills, injections, implants) WO2012000981 discloses a contraceptive kit comprising packaging units comprising 21-28 daily active dosage units. Each daily active dosage unit comprises ≥ 2 mg drospirenone without estrogen.

Combined hormonal contraceptives comprise a combination of a progestin and of an estrogen component.

A number of pharmacologic effects contribute to the contraceptive effects of progestins. These include inhibiting ovulation by suppressing the function of the hypothalamic-pituitary-ovarian axis; modifying the subsequent pituitary surge of luteinizing hormone and follicle-stimulating hormone. It includes also slowing transport of the ovum through the fallopian tubes, which limits the time available for fertilization; thickening cervical mucus, which impedes sperm transit; and inhibiting the activation of spermatic enzymes required for ovum penetration. The primary aim of the estrogen is to provide adequate cycle (bleeding) control; it also inhibits the release of follicle stimulating hormone and consequently prevents follicle development.

One of the adverse effects of combined oral contraceptives is the increased risk of venous thromboembolism (VTE) into two clinical characteristics: deep vein thrombosis or pulmonary embolism. Every year 10,000 women of childbearing age suffer a venous thromboembolic disease and this incidence is increased threefold to fivefold in women who use combined hormonal contraceptives. In women at fertile age, combined hormonal contraception remains the most important factor for the development of VTE and remains in most cases the only factor that triggers the disease (Blanco-Molina MA et al., Progestin-only contraception and venous thromboembolism. Thrombosis Research.2012; 129:e257-e262).

Obesity is an independent risk factor for the development of venous thromboembolism (VTE). (Abdollahi M. et al. Thromb. Haemost. 2003;89 (3):493-498). Use of combined oral contraceptive is associated with increased risk of VTE in obese patients. A population-based case-control study performed in the Netherlands has revealed this increased risk. Among women who did not use oral contraceptives, the risk increased 2.5-fold for overweight women and 3.0-fold for obese women compared to normal weight women who do not use oral contraceptives. Relative to non-users having a normal BMI, oral contraceptive users who were overweight had an 11.6-fold increased risk of VTE and those who were obese had a 23.8-fold increased risk of VTE. (Murthy AS. Semin Reprod Med. 2010 Mar;28(2):156-63 ; Pomp ER et al. Br J Haematol 2007;139:289-296.).

The estrogen component of contraceptive formulations was considered to be mainly responsible for the prothrombotic effect of combined hormonal contraception.

According to guidelines from the World Health Organization and US Centers for Disease Control and Prevention (Guidelines on "Medical eligibility criteria for contraceptive use."), the use of combined oral contraceptives is discouraged by women of 35 years age and older, who are obese, hypertonic, smoke or have diabetes. All modes of progestin-only contraception are advocated, even for higher VTE risk women such as those with hereditary thrombophilia, history of estrogen induced venous thromboembolism, or history of recurrent venous thromboembolism (Centers for Disease Control and Prevention. US medical eligibility criteria for contraceptive use. MMWR Early Release 2010;59:1-86. Department of Reproductive Health WHO. Medical eligibility criteria for contraceptive use. 4th ed. WHO Press, 2009).

It is recalled that progestin-only contraception (POC) relies upon maintaining a high level of progesterone in the female body, hence inhibiting the secretion of the follicle stimulating hormone and the luteinizing hormone. POC treatment results in the absence of development of any new egg follicles.

In POC contraceptive formulations, one may cite numerous progestogen molecules, such as norethindrone, ethynodiol diacetate, levonorgestrel, desogestrel, lynestrenol, drospirenone or depot medroxyprogesterone acetate (DMPA) that have been proven efficient as contraceptive active ingredients.

Regarding progestin-only contraception, a systematic review and meta-analysis from Mantha evaluating the risk of VTE in progestin-only contraceptives users did not identify a significant risk of venous thromboembolism associated with use of progestin-only contraception. (Mantha S et al. BMJ 2012;345:e4944). In a recent study, Lidegaard analyzed more than 29 000 women years for a third generation progestin-only pill (desogestrel) and failed to show any increased VTE risk associated with its use (adjusted venous thromboembolic event rate 0.64 (95% confidence interval 0.29 to 1.42)) (Lidegaard O. et al., 2001-9. BMJ 2011;343:d6423).

Consequently, progestin-only contraceptives should be the best option for women at risk of VTE and thus should be a safe option for overweight or obese women.

However the main known disadvantage of progestin-only contraceptives is the alteration of the bleeding profile, resulting in breakthrough bleeding or spotting during the hormonal treatment.

It is well-known that bleeding irregularities are one of the prominent reasons for discontinuation of contraception, which increases risk of unintended pregnancy. Women discontinuing oral contraceptives often fail to substitute another contraceptive or adopt a less effective method. Indeed, a study has shown that among 6% of users who discontinued oral contraceptives because of irregular bleeding, 23% had unintended pregnancy (Rosenberg MJ et al.. J Reprod. Med. 1995;40(5);355-360).

The prevalence of breakthrough bleeding in overweight or obese women using hormonal contraception is unknown. A retrospective analysis of data from a study evaluating 2893 women (613 were overweight or obese) using two different formulations of a low-dose oral contraceptive found that rates of breakthrough bleeding were not different across weight categories (Hampton RM et al., Contraception 2008;77(6):415-419). Another study showed that during the use of the contraceptive vaginal ring Nuvaring (etonogestrel/ethinyl estradiol), there was overall no difference in the total number of bleeding or spotting days reported by the group of normal BMI women and obese women (normal BMI: 8.7 ± SD 7.3 days, obese: 7.7 ± SD 6.0 days p= 0.64) (Dragoman M. et al., Contraception. 2013 Apr; 87(4): 432-436).

Irregularities in amount and frequency of menstrual flow and weight gain are well documented side effects of POC therapy, such as DMPA, a long acting reversible progestin-only contraceptive birth control drug that is injected every 3 months (Harel Z et al. Adolescents' reasons for and experience after discontinuation of the long-acting contraceptives Depo-Provera and Norplant. J Adolesc Health 1996;19(2):118-23; Kaunitz AM. Injectable depot medroxyprogesterone acetate contraception: an update for U.S. clinicians. Int J Fertil Womens Med 1998;43(2):73-83; Keder LM et al. Compliance with depot medroxyprogesterone acetate: a randomized, controlled trial of intensive reminders. Am J Obstet Gynecol 1998;179(3 Pt 1):583-5; Fraser IS et al.. Depo-Provera use in an Australian metropolitan practice. Med J Australia 1994;160(9):553-6; Nutley T et al. Treatment of bleeding problems associated with progestin-only contraceptives: survey results. Adv Contracept 1997;13(4):419-28; Hill DA. Gynecology case challenge: vaginal bleeding in a woman taking an injectable contraceptive. Medscape Womens Health 1998;3(1):4).

Sole one experimental study relating to DMPA-based POC contraception seemed to show a lower risk of excessive bleeding in overweight women (Connor et al.; J. Am. Borad Fam. Pract. ; 2002, vol. 15(1): 7-10) or obese women (Belsey et al.; Contraception. 1988 Aug;38(2):243-57). Connor et al. (2002) showed that women presenting excessive weight or obesity, when under POC treatment with DMPA, showed a lower risk of increased or excessive bleeding compared with height-weight proportional counterparts. Connor et al. (2002) focused on physiological effects encompassing excessive flow, excessive bleeding, continuous bleeding and continuous flow (globally termed "excessive bleeding" therein) altogether with effects encompassing increased spotting, increased flow, heavier menses and increased duration of menses (globally termed "increased bleeding" therein). The comparative results disclosed by Connor et al. (2002) concern exclusively a unique and global bleeding parameter encompassing both "excessive bleeding" and "increased bleeding".

However, Connor et al. were silent on the possible effect of DMPA on the duration of the bleeding events, and especially on the number of days for which the bleeding or spotting occurs.

Nevertheless, it flows from the study of Connor et al. that women presenting excessive weight or obesity still present unwanted spotting and bleeding.

Still further, it may also be referred to a study published by Casey et al. (2013, Contraception, Vol. 87 : 370-374) relating to etonogestrel-based POC contraceptive under the form of a subdermal implant (ESI). Casey et al. (2013) showed that obese women were less likely to have the ESI implant removed for bleeding irregularities compared to non-obese women.

It flows from the prior art knowledge discussed above that each known progestogen candidate molecule aimed at formulating POC contraceptive formulations adapted to specific administration regimen may behave differently, including regarding their effect on various kinds of bleeding events.

Finally, it is generally known in the art that POC treatment is also often correlated with a weight gain, which may become challenging for women with excess weight, in particular overweight women, and especially for obese women, which weight gain effect may enhance the occurrence of diseases linked to overweight and/or obesity, such as, e.g., coronary heart diseases, high blood pressure, stroke, type 2 diabetes, abnormal blood fats, metabolic syndrome, cancer, osteoarthritis, sleep apnea, obesity hypoventilation syndrome, infertility, gallstones, gout.

Specifically, a systematic review from Curtis, looking at the use of progestin-only contraceptives in obese women, suggested that adolescent DMPA users who are obese or overweight will gain more weight than normal weight users (Curtis KM. et al. Progestogen-only contraceptive use in obese women. Contraception 2009;80(4):346-354). Women often blame contraception for their weight gain. Many women will discontinue their contraception because of this perceived side effect. Indeed, studies of oral contraceptive users have found that a perceived weight gain is one of the leading reasons for discontinuation in US women (Rosenberg M. Weight change with oral contraceptive use and during the menstrual cycle: results of daily measurements. Contraception 1998;58:345-9)

Therefore, due to the drawbacks mentioned above, occurrence of unwanted bleeding or spotting, weight gain, women with excess weight, in particular overweight women, and especially obese women, are more likely to drop off POC treatment, hence increasing their chances of unintended pregnancy.

Therefore, there is a need to provide women with excess weight, which encompass overweight and obese women, with safe and efficient contraceptive formulations, especially with better-tolerated contraceptive formulations showing improved patient compliance.

### SUMMARY OF THE INVENTION

This invention relates to the use of drospirenone (DRSP) as the sole contraceptive ingredient comprised in a daily active dosage unit in an amount of at least 3 mg as a contraceptive for a female patient affected with obesity, as featured by a BMI of 30 kg/m² or greater.

The invention is as defined in the claims. The references to methods of treatment in the summary and detailed description of the invention in this description are to be interpreted as references to the compounds, pharmaceutical compositions and medicaments of the present invention for use in a method for treatment of the human body by therapy.

This disclosure also relates to the use of drospirenone in the manufacture of a contraceptive formulation which leads to a reduction in the number of days with bleeding events in female patients affected with excess weight, which includes overweight female patients and obese female patients. The general and substantial reduction in the number of days with bleeding events, as compared with female patients who are not affected with excess weight, is disclosed throughout the present specification. The said contraceptive formulation, which contains drospirenone as the sole contraceptive ingredient, is described in detail and in its various embodiments throughout the present specification.

The disclosure also relates to a method comprising administering a pharmaceutical composition comprising drospirenone in an amount of at least 3 mg to a female patient affected with excess weight, which encompasses a female patient affected with overweight as well as a female patient affected with obesity.

This disclosure also pertains to a method comprising administering a pharmaceutical composition comprising drospirenone in an amount of at least 3 mg to a female patient affected with excess weight, which encompasses a female patient affected with overweight as well as a female patient affected with obesity, wherein said pharmaceutical composition allows for a 28 day daily dosing regimen, and wherein after initial administration of said drospirenone has established its contraceptive effect in a patient, said patient may skip up to 4 doses within 28 day daily dosing regimen period.

The disclosure also concerns the use of drospirenone in the manufacture of a contraceptive formulation which leads to a general substantial reduction of the number of days with bleeding events in female patients, and especially female patients affected with excess weight, which encompasses female patients affected with obesity. The general and substantial reduction in the number of days with bleeding events, as compared with female patients who are not affected with excess weight, is disclosed throughout the present specification. The said contraceptive formulation, which contains drospirenone as the sole contraceptive ingredient, is described in detail and in its various embodiments throughout the present specification.

The disclosure also pertains to the use of drospirenone in the manufacture of a contraceptive kit which leads to a general substantial reduction of the number of days with bleeding events in female patients, wherein said contraceptive kit comprises one or more packaging units wherein each packaging unit comprises 21 to 28 daily active dosage units and wherein:
a) each daily active dosage unit comprises drospirenone in an amount of at least 3 mg, without estrogen, and
b) a single daily active dosage unit, when orally administered under fasting conditions, is adapted to provide a pharmacokinetic profile for drospirenone having:
   (i) a mean tₘₐₓ ranges from 2.2h to 6h and
   (ii) a mean Cₘₐₓ which is less than about 30 ng/ml, and
   (iii) optionally a mean AUC0h-tlast of at least about 300 ng*h/ml..

The Cₘₐₓ and tₘₐₓ values refer to the maximum DRSP plasma concentration and the time to reach it, respectively, after the oral administration of a single daily dosage unit of the DRSP-containing composition of interest. In other words, Cₘₐₓ and tₘₐₓ refer to the characteristics of drospirenone plasma concentration peak observed after the oral intake of a single daily dosage unit of the composition of interest.

The AUC₀ₕ₋ₜₗₐₛₜ corresponds to the area obtained by integration of the drospirenone plasma concentration versus time over the interval [0h-tlast], the point "0h" referring to the oral intake of a single daily dosage unit of the composition of interest and the point "tlast" refers to the last time for which plasma concentration of DRSP can be quantifiable.

DRSP plasma concentration may be determined by well-known methods. For example, an appropriate method of quantification comprises the extraction of DRSP from human plasma and then its quantification using liquid chromatography coupled with tandem mass spectrometry.

In an embodiment, one skilled in the art may adapt the analytical method described by Kirk et al (Rapid Communication in Mass Spectrometry, 2006; 20:1247-1252). Such a method comprises a step of derivatization of drospirenone with Girard P hydrazine solution in order to increase the response of DRSP during the subsequent MS analysis. This method is generally appropriate to quantify DRSP in human EDTA plasma over a concentration range from about 0.25 to about 100 ng/ml.

As used herein, the mean AUC₀ₕ₋ₜₗₐₛₜ, the mean Cₘₐₓ and the mean tₘₐₓ refer to arithmetic mean values determined from individual pharmacokinetic data obtained for a group of healthy female volunteers of child-age bearing subjected to a single oral administration of one daily dosage unit of a drospirenone-containing composition. The group of healthy female volunteers may comprise enough women to provide statistically confident pharmacokinetic results. Preferably, the said group comprises at least ten healthy women of child-bearing age.

As used herein, a healthy woman of child-bearing age refers to a premenopause Caucasian female between 18 and 40 years, with normal body weight and with no health problem, in particular, with no metabolism, renal, liver or gynecologic disorders.

The disclosure also concerns the use of drospirenone in the manufacture of a contraceptive kit which leads to a reduction of the number of days with bleeding events in female patients, wherein said contraceptive kit comprises one or more packaging units wherein each packaging unit comprises 21 to 28 daily active dosage units and wherein:
(a) the amount of drospirenone in each daily active dosage unit is at least 3 mg without estrogen, and
(b) each daily active dosage unit comprises drospirenone in a form such that:
   (i) no more than 50% of the drospirenone initially present in the said daily active dosage unit is dissolved within 30 minutes and
   (ii) at least 50% of said drospirenone is dissolved in a time range from 3 hours to 4 hours, when the daily active dosage is subjected to an *in vitro* dissolution test according to USP XXIII Paddle Method, the percentages of drospirenone being related to the amount of drospirenone initially present in the said daily active dosage unit.

### LEGEND OF THE FIGURES

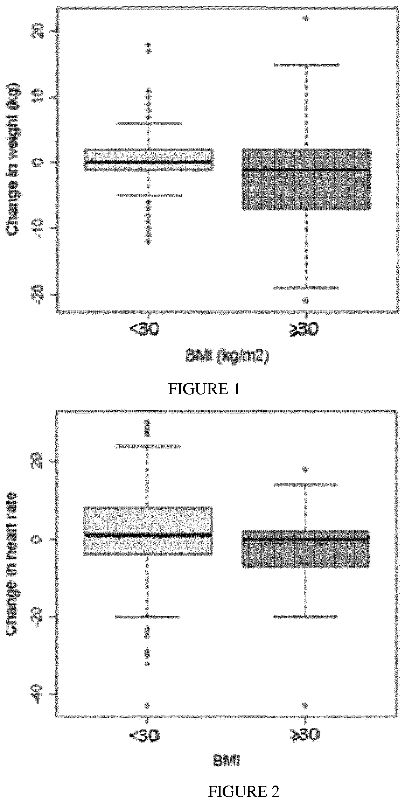

**Figure 1****:** plot illustrating the correlation between the body mass index (BMI) of women undertaking a DRSP-based contraceptive treatment with respect to the change in weight (kg). Change of weight is measured by subtracting the weight at visit 6 (day 24±2 of the 13^{th} cycle) from the weight at screening. Any negative result is indicative of a weight loss. Women with a BMI lower than 30 kg/m² (left) do not present any average weight change, whereas women with a BMI of 30 kg/m² or greater (right) do present a slight but significant change in weight during the course of the contraceptive treatment.
**Figure 2****:** plot illustrating the correlation between the body mass index (BMI) of women undertaking a DRSP-based contraceptive treatment with respect to the change in heart rate. Change in heart rate is measured by subtracting the heart rate at visit 6 (day 24±2 of the 13^{th} cycle) from the heart rate at screening. Any positive result is indicative of a higher heart rate during the course of the contraceptive treatment. Women with a BMI lower than 30 kg/m² (left) do present a slight but significant increase in the heart rate during the course of the contraceptive treatment, whereas women with a BMI of 30 kg/m² or greater (right) do not present any change in the heart rate during the course of the contraceptive treatment.

### DETAILED DESCRIPTION OF THE INVENTION

The inventors have recently developed a new contraceptive kit and a new contraceptive pharmaceutical composition based on drospirenone (DRSP) in a crystallized and non-micronized form (WO 2012/000981). DRSP is a fourth generation of progestogen, which derives from spironolactone and has a pharmacological profile that mimics natural progesterone.

DRSP is devoid of androgenic, glucocorticoid and anti-glucocorticoid activity but does possess potent anti-mineralocorticoid and anti-androgenic properties. It was shown that oral daily doses of at least 3 mg of DRSP are able to inhibit ovulation over a single treatment cycle of 21 days. The combination of 3 mg DRSP /30 µg ethinylestradiol provides a reasonable contraceptive safety margin by inhibiting ovulation with a low frequency of follicular maturation (Rosenbaum et al., 2000, The European Journal of Contraception and Reproductive Health Care, 5,16-24).

Contrarily to other DRSP-based contraceptive formulations available on the market, DRSP-based contraceptive formulation according to WO 2012/000981 relies upon a slow dissolution rate of DRSP *in vitro.*

Moreover, the DRSP-based contraceptive formulation according to WO 2012/000981 displays a peculiar pharmacokinetic profile. In particular, a single daily active dosage unit comprising DRSP in an amount of at least 3 mg, when orally administered under fasting conditions, is adapted to provide a pharmacokinetic profile for DRSP having:
- a mean tₘₐₓ ranging from 2.2 h to 6h and
- a mean Cₘₐₓ which is less than about 30 ng/ml.

The contraceptive pharmaceutical composition based on drospirenone (DRSP) in a crystallized and non-micronized form disclosed in WO 2012/000981 has been suggested being suitably administered to female patients having a BMI lower than 30 kg/m².

Within the scope of the following terminology is being used for a standardized description of bleeding and/or spotting in female patients under contraceptive treatment (Mishell et al., 2007, Contraception, 75(1):11).

The term "bleeding" or the expression "bleeding event" is intended to refer to a blood loss that requires the use of a tampon, pad or panty liner.

The term "spotting" or the expression "spotting event" is intended to refer to a minimal blood loss that does not require use of any type of protection.

The expression "episode of bleeding and/or spotting" is intended to refer to bleeding and/or spotting days bounded on either end by two days of no bleeding or spotting.

In some embodiment, the term "bleeding" may also encompass "spotting".

As it is shown in the examples herein, the number of days with bleeding events per treatment cycle in women with excess weight, i.e. in women having a BMI of 25 kg/m² or more, who are treated with the DRSP-based POC formulation described herein (i) is reduced by about 22.5% as compared with non-overweight women subjected to the same contraceptive treatment, i.e. women having a BMI of 24.9 kg/m² or less, during the treatment cycles 2 to 4, (ii) is reduced by about 27.7% as compared with non-overweight women subjected to the same contraceptive treatment, i.e. women having a BMI of 24.9 kg/m² or less, during the treatment cycles 2 to 6, (iii) is reduced by about 30.8% as compared with non-overweight women subjected to the same contraceptive treatment, i.e. women having a BMI of 24.9 kg/m² or less, during the treatment cycles 2 to 9, (iv) is reduced by about 23.1% as compared with non-overweight women subjected to the same contraceptive treatment, i.e. women having a BMI of 24.9 kg/m² or less, during the treatment cycles 5 to 7 and (v) is reduced by about 22.5% as compared with non-overweight women subjected to the same contraceptive treatment, i.e. women having a BMI of 24.9 kg/m² or less, during the treatment cycles 7 to 9. The difference observed is statistically significant (p<0.01) using the Wilcoxon-rank sum test.

As it is further shown in the examples herein, the average number of days of bleeding events in women with excess weight, i.e. in women having a BMI of 25 kg/m² or more, (i) does not exceed 13% per treatment cycle, during the treatment cycles 2 to 4; (ii) does not exceed 11% per treatment cycle during the treatment cycles 2 to 6; (iii) does not exceed 10% per treatment cycle, during the treatment cycles 2 to 9, during the treatment cycles 5 to 7, or during the treatment cycles 7 to 9.

As used herein, "a treatment cycle" encompasses a 28 days period of treatment, wherein said treatment comprises the administration of 21 to 28 consecutive daily doses of the active test product tablets and may comprise at most the administration of 7 daily doses of placebo tablets.

In a preferred embodiment, the treatment cycle comprise the administration of 24 consecutive daily doses of the active test product tablets and subsequently 4 consecutive days of administration of placebo tablets.

As used herein, "the number of days with bleeding events per treatment cycle" encompasses the number of days with one or more bleeding episode during a cycle of treatment, irrespective the time period of the cycle, i.e. active test product or placebo.

As shown in the examples herein, the number of days with bleeding events per treatment cycle in women with excess weight, i.e. in women having a BMI of 25 kg/m² or more, who are treated with the DRSP-based POC formulation described herein is always reduced by at least 5.0% as compared with women with no excess weight subjected to the same contraceptive treatment, i.e. women having a BMI of 24.9 kg/m² or less, during any cycle of the treatment cycles studied.

As used herein, a reduction of the number of days with bleeding events per treatment cycle by "at least 5%" encompasses a reduction by at least 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% and 30%.

For example, the mean percentage of reduction of the number of days with bleeding events per treatment cycle in women with excess weight treated with the DRSP-based POC formulation as compared with women with no excess weight subjected to the same contraceptive treatment, may be evaluated for 3 consecutive cycles of treatment and may range from 15% to 30%, preferably from 20% to 25%, more preferably from 22% to 24%.

It is to be understood that the percentage of reduction of the number of days with bleeding events per treatment cycle in women with excess weight may be submitted to variations, depending on parameters such as e.g. initial weight, age, time elapsed from the beginning of the treatment.

As it is further shown in the examples herein, the number of days with bleeding events per treatment cycle in obese women, i.e. in women having a BMI of 30 kg/m² or more, who are treated with the DRSP-based POC formulation described herein (i) is reduced by about 60.1% as compared with non-obese women subjected to the same contraceptive treatment, i.e. women having a BMI of 29.9 kg/m² or less, during the treatment cycles 2 to 4, (ii) is reduced by about 67.9% as compared with non-obese women subjected to the same contraceptive treatment, i.e. women having a BMI of 29.9 kg/m² or less, during the treatment cycles 2 to 6, (iii) is reduced by about 69.7% as compared with non-obese women subjected to the same contraceptive treatment, i.e. women having a BMI of 29.9 kg/m² or less, during the treatment cycles 2 to 9, (iv) is reduced by about 48.1% as compared with non-obese women subjected to the same contraceptive treatment, i.e. women having a BMI of 29.9 kg/m² or less, during the treatment cycles 5 to 7 and (v) is reduced by about 62.0% as compared with non-obese women subjected to the same contraceptive treatment, i.e. women having a BMI of 29.9 kg/m² or less, during the treatment cycles 7 to 9.

The difference observed is statistically significant (p<0.05) using the Wilcoxon-rank sum test.

As it is further shown in the examples herein, the average number of days of bleeding events in obese women, i.e. in women having a BMI of 30 kg/m² or more, (i) does not exceed about 7% during the treatment cycles 2 to 4, during the treatment cycles 5 to 7; (ii) does not exceed about 5% during the treatment cycles 2 to 6, during the treatment cycles 2 to 9, during the treatment cycles 7 to 9.

In addition, as it is further shown in the examples herein, as compared to other POC-based contraceptive composition, the drospirenone-based composition may benefit obese women, since a significant decrease in the number of bleeding or spotting events during the period cycles was observed.

Thus, as shown in the examples herein, the number of days with bleeding events per treatment cycle in obese women, i.e. in women having a BMI of 30 kg/m² or more, who are treated with the DRSP-based POC formulation described herein is always reduced by about 10.0% or more as compared with non-obese women subjected to the same contraceptive treatment, i.e. women having a BMI of 29.9 kg/m² or less, during any cycle of the treatment cycles studied.

As used herein, a reduction of the number of days with bleeding events per treatment cycle by "at least 10%" encompasses a reduction by at least 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% and 50%.

The mean percentage of reduction of the number of days with bleeding events per treatment cycle in obese women treated with the DRSP-based POC formulation as compared with non-obese women subjected to the same contraceptive treatment, may further be evaluated for 3 consecutive cycles of treatment and may range from 30% to 75%, preferably from 40% to 75%, more preferably from 45% to 65%.

The reduction in the number of days with bleeding or spotting that is shown by the inventors may benefit to women with excess weight, which includes overweight and obese women, and ameliorate considerably their living comfort, while providing a compliant contraceptive treatment, and thus an increased efficiency in the contraceptive treatment.

In addition to the reduction of the number of days with bleeding or spotting, oral administration of a DRSP-based POC treatment according to WO 2012/000981 to women with excess weight, in particular overweight women, and especially to obese women, may also provide a benefit on the weight change together with a benefit on the resting heart rate change, as shown in the examples herein.

These surprising observations could not have been expected from the previous clinical evaluation disclosed in WO 2012/000981.

Indeed, such DRSP-based contraceptive composition, when administered to women with excess weight, in particular overweight women, and especially to obese women, results in an average significant weight loss, and no change to the resting heart rate as compared to women from the general population, for which, no effect on weight change and a slight significant increase of the resting heart rate is observed during the course of this treatment.

Therefore, the DRSP-based contraceptive treatment described herein allows women with excess weight, including women affected with obesity, which encompasses women having a body mass index (BMI) of 30 kg/m² or greater, to better comply with the prescribed contraceptive treatment, thereby reducing the risks of dropping off the said contraceptive treatment and therefore increasing the risks of unintended pregnancy.

Thus, in some embodiments, the present disclosure relates to drospirenone as the sole active ingredient comprised in a daily active dosage unit in an amount of 3 mg or more, and especially in an amount ranging from 3 to 6 mg, for use as a contraceptive for reducing the number of days with bleeding events in overweight women by 5% or more, and preferably by 10% or more, as compared with non-overweight women subjected to the same contraceptive treatment.

Further, the experimental data disclosed herein show that the effect of reduction of the number of days with bleeding events per treatment cycle tends to increase with the duration of the contraceptive treatment with the DRSP-POC formulation, to reach a reduction of 15% or more. As disclosed in the examples herein, a reduction of more than 30% of the days with bleeding events per treatment cycle occurs in overweight women during the time period of the treatment cycles 2 to 9.

Still further, the present invention relates to the use of drospirenone as the sole active ingredient comprised in a daily active dosage unit in an amount of 3 mg or more, and especially in an amount ranging from 3 to 6 mg, as a contraceptive for reducing the number of days with bleeding events in obese women, as featured by a BMI of 30 kg/m² or greater, by 10% or more as compared with non-obese women subjected to the same contraceptive treatment. As disclosed in the examples herein, a reduction of more than 45% of the days with bleeding events occurs in obese women during the time period of the treatment cycles 2 to 9.

As it is further shown in the examples herein, the number of days of bleeding events in an overweight or obese patient does not exceed about 20%, 15%, 10%, 8%, or 5% in any treatment cycle subsequent to an initial treatment cycle.

### 1- Daily active dosage of drospirenone

In one aspect, the invention relates to the use of DRSP as the sole contraceptive ingredient comprised in a daily active dosage unit in an amount of at least 3 mg as a contraceptive for a female patient affected with obesity, as featured by a BMI of 30 kg/m² or greater.

As used herein, by "contraceptive ingredient" is meant an ingredient that may prevent pregnancy when daily administered in an effective amount to a female patient over a period of 21 to 28 consecutive days. The contraceptive ingredient may prevent pregnancy to occur by various biological effects. For example, the pregnancy may be prevented by the inhibition of ovulation, by the thickening of cervical mucus (which reduces the sperm viability and penetration) and/ or by prevent embryo implantation.

As used herein, the expression "sole contraceptive ingredient" is intended to mean that DRSP is the unique ingredient that promotes contraception. In other words, the contraceptive ingredient does not comprise an estrogen.

Drospirenone or DRSP, namely 6β, 7β,15β, 16β-dimethylen-3-oxo-17a-pregn4-ene-21,17-carbo-lactone, and further identified by the CAS registry Number 67392-87-4, is a synthetic progestogen with a pharmacological profile very closely related to that of natural progesterone.

As used therein, the term "drospirenone" refers to drospirenone itself, or a solvate drospirenone.

DRSP may be prepared by well-known methods described in the prior art, for example, described in US 4129564, WO9806738, EP11746101 or WO2006061309. The method described in WO2006061309 may be particularly suitable for preparing DRSP.

It goes without saying that the method for preparing DRSP may be performed so as to meet the Good manufacturing practice (GMP) requirements.

To ensure a good bioavailability of DRSP, a significant amount of the DRSP initially comprised in the contraceptive composition has to be released in a reasonable time range.

A good bioavailability of DRSP may be achieved in the case of compositions comprising DRSP which had an *in vitro* dissolution rate of DRSP such that at least 50% of the DRSP initially present in the said compositions was dissolved in a time range from 3 hours to 4 hours.

As used herein, "an active daily dosage unit" means a dosage unit which is able to prevent pregnancy when daily administered to a female patient. In preferred embodiments, the active daily dosage unit is able to inhibit ovulation.

As used herein, a "female patient" refers to a female individual of child-bearing age i.e. from the puberty to the menopause. Female individual of child-bearing age also include women in peri-menopause.

Body mass index (BMI) is a simple index of weight-for-height that is commonly used to classify overweight and obesity in adults. It is defined as a person's weight in kilograms divided by the square of his height in meters (kg/m²).

The WHO (World Health Organization) accepted weight standard status categories associated with BMI ranges for adults are as follows:
- a BMI below 18.5 kg/m² is indicative of an underweight status;
- a BMI ranging from 18.5 kg/m² to 24.9 kg/m² is indicative of a normal weight status;
- a BMI ranging from 25.0 kg/m² to 29.9 kg/m² is indicative of an overweight status; and
- a BMI of 30.0 kg/m² or greater is indicative of an obesity status.

As used herein, a female patient affected with excess weight, encompasses a female patient having a BMI of 25.0 kg/m² or more, which includes an overweight female patient and an obese female patient.

As used herein, an overweight female patient includes a female patient having a BMI ranging from 25.0 kg/m² to 29.9 kg/m².

As used herein, an obese female patient, or a female patient affected with obesity, encompasses a female patient having a BMI of 30.0 kg/m² or more.

In certain embodiments, the female patient has a BMI of 25.0 kg/m² or greater.

In certain preferred embodiments, the female patient has a BMI of 30.0 kg/m² or greater.

In certain embodiments, the said daily active dosage unit is comprised within a contraceptive kit comprising one or more packaging units wherein each packaging unit comprises 21 to 28 daily active dosage units and wherein:
(a) the amount of DRSP in each daily active dosage unit is at least 3 mg without estrogen, and
(b) each daily active dosage unit comprises DRSP in a form such that:
   (i) no more than 50% of the DRSP initially present in the said daily active dosage unit is dissolved within 30 minutes and
   (ii) at least 50% of the said DRSP is dissolved in a time range from 3 hours to 4 hours,
when the daily active dosage unit is subjected to an *in vitro* dissolution test according to the USP XXIII Paddle Method, the percentages of DRSP being related to the amount of DRSP initially present in the said daily active dosage unit.

The daily active dosage unit comprising DRSP is characterized by a slow dissolution rate of DRSP *in vitro.*

As used herein, by "a slow dissolution rate of DRSP *in vitro"* is meant that the release of DRSP is such that no more than 50% of DRSP initially present in the said daily active dosage unit is dissolved within 30 minutes when the said daily active dosage unit is subjected to a dissolution test.

As intended herein, no more than 50% of the DRSP encompasses no more than 45%, 40%, 35%, 30%, 25%, 20%, 15%, 10% of the DRSP initially present in the daily active dosage unit.

In some embodiments, no more than 40% of the DRSP initially present in the daily active dosage unit is dissolved within 30 min.

As used herein, the percentage of DRSP is related to the amount of DRSP initially present in the said daily active dosage unit.

The *in vitro* dissolution rate of DRSP may be assessed by anyone of well-known methods described in the prior art.

The *in vitro* dissolution rate of DRSP is preferably assessed by the USP XXIII Paddle Method. Briefly, a tablet consisting of the contraceptive composition comprising DRSP to be tested is placed in 900 mL of water at 37°C (± 0.5°C). The dissolution test is performed using a USP dissolution test apparatus 2 at a stirring rate of 50 rpm.

In a preferred embodiment, amount of DRSP in each daily active unit dosage is at least 3 mg of DRSP. At least 3 mg of DRSP encompasses at least 3.5 mg, at least 4 mg of DRSP, at least 4.5 mg of DRSP, at least 5 mg or at least 5.5 mg of DRSP.

In some embodiments, the active daily dosage unit which consists of the contraceptive composition as describes above may comprise a DRSP amount ranging from about 3 mg to about 6 mg. A daily dose ranging from about 3 mg to about 6 mg encompasses daily doses of 3.0 mg, 3.5 mg, 4.0 mg, 4.5 mg, 5.0 mg, 5.5 mg and 6 mg.

In certain embodiments, the amount of DRSP in each daily active unit dosage ranges from about 3.5 mg to 4.5 mg.

As used herein, the term "about" before a "specific value" defines a range from "the specific value minus 10% of the specific value" to "the specific value plus 10% of the specific value". For example, "about 50" defines a range from 45 to 55.

In certain embodiments, the one or more packaging units further comprise from 1 to 7 daily dosage units of a pharmaceutically acceptable placebo.

Within the scope of the instant disclosure, the term "placebo" is intended to mean a pharmacologically inert or innocuous substance.

In certain embodiments, each packaging unit comprises 24 daily active dosage units.

In certain embodiments, each packaging unit comprises 4 daily placebo dosage units.

In certain embodiments, DRSP is in a crystalline form.

In certain embodiments, DRSP is in a non-micronized form.

As mentioned already, DRSP in a crystalline and non-micronized form allows for a slow dissolution rate of DRSP *in vitro.*

One way to obtain the DRSP-containing composition described herein is to use DRSP in a particle form, such as a non-micronized particle form, having an appropriate specific surface area. It has been also shown that DRSP in a particle form having a specific surface area from about 2000 cm²/g to about 8500 cm²/g may be suitable for obtaining the contraceptive compositions described herein. The specific surface area may be experimentally determined using the BET method (gas adsorption method).

In certain embodiments, DRSP in a particle form has a specific surface area from about 2,000 cm²/g to about 8,500 cm²/g.

Such a specific area range which includes values of about 2000 cm²/g, 2500 cm²/g, 3000 cm²/g, 3500 cm²/g, 4000 cm²/g, 4500 cm²/g, 5000 cm²/g, 5500 cm²/g, 6000 cm²/g, 6100 cm²/g, 6200 cm²/g, 6300 cm²/g, 6400 cm²/g, 6500 cm²/g, 6600 cm²/g, 6700 cm²/g, 6800 cm²/g, 6900 cm²/g, 7000 cm²/g, 7500 cm²/g, 8000 cm²/g and 8500 cm²/g.

Concerning the size particle distribution, DRSP particles having a diameter greater than 200 µm shall be avoided in order to not drastically impair the *in vitro* dissolution rate and, thus, the *in vivo* bioavailability since such particles are poorly soluble.

In certain embodiments, DRSP has a d₅₀ of less than 70 µm.

In certain preferred embodiments, the d₅₀ of the DRSP particles ranges from 10 µm to 60 µm. A d₅₀ ranges from about 10 µm to about 60 µm encompasses a d₅₀ of 10 µm, of 15 µm, of 20 µm, of 25 µm, of 30 µm, of 35 µm, of 40 µm, of 45 µm, of 50 µm, of 55 µm and of 60 µm.

In some embodiments, the particle size distribution of the DRSP present in the composition is characterized by:
(i) a d₉₀ particle size less than about 100 µm, and/or
(ii) a d₅₀ particle size ranging from about 10 µm to about 60 µm and/or
(iii) a d₁₀ particle size more than about 3 µm .

In some other embodiments, the d₅₀ of DRSP particles ranges from about 10 µm to about 30 µm. In such embodiments, the particle size distribution of the DRSP present in the composition is characterized by at least one of the following characteristics:
(i) a d₉₀ particle size less than about 100 µm,
(ii) a d₅₀ particle size ranging from about 10 µm to about 30 µm and
(iii) a d₁₀ particle size more than about 3 µm.

As used herein, by "d₉₀ particle size" is meant that the particle size distribution is such that at least 90% of the particles have a particle size diameter of less than the specified value.

As used herein, by "d₅₀ particle size" is meant that the particle size distribution is such that at least 50% of the particles have a particle size diameter of less than the specified value.

As used herein, by "d₁₀ particle size" is meant that the particle size distribution is such that at least 10% of the particles have a particle size diameter of less than the specified value.

d90 particle size less than about 100 µm include d₉₀ particle sizes less than about 90 µm, 80 µm, 70 µm, 60 µm, 55 µm, 50 µm, 45 µm, 40 µm, 38 µm, 36 µm, 34 µm, 32 µm, 30 µm, 28 µm, 26 µm, 24 µm, 22 µm, 20 µm.

The d₅₀ particle size values ranging from about 10 µm to about 30 µm include values of about 10 µm, 11 µm, 12 µm, 13 µm, 14 µm, 15 µm, 16 µm, 18 µm, 19 µm, 20 µm, 21 µm, 22 µm, 23 µm, 24 µm, 25 µm, 26 µm, 27 µm, 28 µm, 29 µm, 30 µm.

The d₁₀ particle size values more than about 3 µm include d10 particle size values more than about 3 µm, 3.5 µm 4.5 µm, 5 µm, 6 µm, 7 µm, 8 µm, 9 µm, 10 µm, 11 µm, 12 µm.

It goes without saying that d₁₀ particle size value is smaller than d₅₀ particle size value which is smaller than d₉₀ particle value.

The DRSP particle size distribution, in particular d₉₀, d₁₀ and d₅₀ values, may be determined by well-known methods of the prior art such as sieve analysis, laser diffraction methods, photo-analysis or optical counting methods. Laser diffraction methods are particularly preferred. The particle size distribution may be determined by laser diffraction in wet dispersion. The dispersant is preferably water.

In some embodiments, the pharmaceutical composition comprises DRSP in a particle form having a particle size distribution having a combination of two characteristics selected from:
(i) a d₉₀ particle size less than about 100 µm,
(ii) a d₅₀ particle size ranging from about 10 µm to about 30 µm and
(iii) a d₁₀ particle size more than about 3 µm.

In other words, the particle size distribution of DRSP display a combination of characteristics selected from characteristic (i) and characteristic (ii), characteristic (i) and characteristic (iii), and, characteristic (ii) and characteristic (iii).

In some other embodiments, the pharmaceutical composition comprising DRSP in a non-micronized form having a particle size distribution characterized in that:
(i) d₉₀ particle size is less than about 100 µm,
(ii) d₅₀ particle size ranging from about 10 µm to about 30 µm and
(iii) d₁₀ particle size is more than about 3 µm

In a preferred embodiment, the DRSP particle distribution is further characterized in that d₉₀ particle size value is less than 50 µm and in that no particle has a size greater than 80 µm.

In some embodiments, the contraceptive composition comprises DRSP in a particle form having a d₉₀ particle size which ranges from about 20 µm to about 40 µm, a d₅₀ particle size which ranges from about 10 µm to about 30 µm and a d10 which ranges from about 3 µm to about 9 µm and wherein no particle has a size greater than 80 µm, more preferably no particle has a size greater than 60 µm.

In some other embodiments, the contraceptive composition comprises drospirenone in a particle form having
(i) a d₉₀ particle size which ranges from about 30 µm to about 40 µm,
(ii) a d₅₀ particle size which ranges from about 15 µm to about 25 µm and
(iii) a d₁₀ which ranges from about 5 µm to about 9 µm and wherein no particle has a size greater than 80 µm, more preferably no particle has a size greater than 60 µm.

In some other embodiments, the contraceptive ingredient is represented by DRSP in a particle form having a specific surface area from about 2000 cm²/g to about 8000 cm²/g and having a d₅₀ particle size ranges from 10 µm to 60 µm.

To obtain DRSP in a particle form having the specific surface area and/or the particle size distribution as described above, the one skilled in the art may use well-known methods of the prior art such as milling process optionally combined with sieve process.

For example, DRSP, obtained by anyone of the synthesis methods described in the prior art, may be subjected to ball mill or hammer mill step optionally followed by a vibrating sieve steps. The subsequent vibrating sieve steps may remove finest and biggest particles of DRSP which would impair the pharmacokinetic profile and the *in vitro* dissolution profile of DRSP.

The one skilled in the art may adjust the parameters of the milling and sieve steps by routine experiments to obtain the appropriate particle form of DRSP. Appropriate mills which may be used include fluid energy mill, ball mill or rod mill, hammer mill, cutting mill and oscillating granulator.

An appropriate particle form of DRSP may be also prepared by crystallisation or precipitation process optionally combined with a sieve step in order to fully control the size of DRSP particles. The precipitation process may comprise the steps of (i) dissolving DRSP in a water-miscible solvent and then (ii) dispersing the resulting solution in cold water under stirring so that to induce the precipitation of DRSP. The DRSP particles may be then recovered by a filtration process.

The water-miscible solvents may be a solvent commonly used in crystallisation or precipitation process such as methanol, ethanol, isopropanol, dimethylformamide, tetrahydrofuran, dioxane or dimethyl sulfoxide, dimethylacetamide or acetone.

Such a process enables to obtain DRSP essentially in crystallized form.

By routine experiments, the one skilled in the art may determine the parameters of the precipitation process to be used so as to obtain the appropriate form of DRSP.

The one skilled in the art may adjust the parameters of the said precipitation process (such as the amounts of solvent, of water and optionally that of surfactant to be used) by routine experiments.

### 2- Contraceptive method

The disclosure also relates to a method comprising administering a pharmaceutical composition comprising DRSP in an amount of at least 3 mg to a female patient affected with excess weight, wherein said pharmaceutical composition allows for a 28 day daily dosing regimen, and wherein after initial administration of said DRSP has established its contraceptive effect in a patient, said patient may skip up to 4 doses within 28 day daily dosing regimen period.

In some embodiments, the disclosure also relates to a method comprising administering a pharmaceutical composition comprising DRSP in an amount of at least 3 mg to a female patient affected with obesity, wherein said pharmaceutical composition allows for a 28 day daily dosing regimen, and wherein after initial administration of said DRSP has established its contraceptive effect in a patient, said patient may skip up to 4 doses within 28 day daily dosing regimen period.

In some other embodiments, the disclosure also relates to a method comprising administering a pharmaceutical composition comprising DRSP in an amount of at least 3 mg to a female patient having a BMI of 30 kg/m² or greater, wherein said pharmaceutical composition allows for a 28 day daily dosing regimen, and wherein after initial administration of said DRSP has established its contraceptive effect in a patient, said patient may skip up to 4 doses within 28 day daily dosing regimen period.

In some embodiments of the method above, the said female patient has a BMI of 25 kg/m² or greater.

In some embodiments of the method above, the said female patient is affected with obesity.

In certain embodiments, the female patient is having a BMI of 30 kg/m² or greater.

As used herein, "daily dosing regimen" means that the contraceptive method for a female patient affected with obesity comprises the step of administering active daily dosage units consisting of a pharmaceutical composition as fully-described herein to the said female patient over a period of several consecutive days over a period of 28 days, i.e. a period corresponding to the average length of a menstrual cycle.

As used herein, "an active daily dosage unit" means a dosage unit which is able to prevent pregnancy when daily administered to a female patient over a period of 28 consecutive days.

After DRSP has established its contraceptive effect, the method may comprise a second phase that is a free-contraceptive period i.e. a phase during which no contraceptive ingredients is administered to the female patient. During the said second phase, daily placebo dosage units may be administered to the female patient. In some other cases, no pill is administered to the female patient.

By "daily placebo dosage unit" it is understood that the ingredient comprised in said dosage unit is pharmaceutically inert or innocuous. In other words, the daily placebo dosage unit does not contain any contraceptive ingredient, as defined herein.

Such a second phase may enable regular menstrual bleedings to occur and thus may enable to mimic the natural menstrual cycle.

Moreover, the said second phase is believed to enable the secretion of endogenous estradiol which may have some benefits on bone metabolism of the female patient.

In certain embodiments, said pharmaceutical composition further allows during said 28 days daily dosing regimen for said patient to skip up to two non-consecutive days of said DRSP, provided said DRSP skipped dose is taken within about 24 hrs after said up to two skipped non-consecutive days.

In certain embodiments, said skipped up to 4 doses are on non-consecutive days.

In certain embodiments, said skipped up to 4 doses are on consecutive days.

In a general aspect, non-micronized and crystallized form DRSP is preferably used for preparing the pharmaceutical composition.

The daily dosing regimen of DRSP to be administered to a female patient affected with obesity may also be adjusted depending on individual factors such as the age, the body weight, the general health and the diet of the female patient. The said daily dosing regimen may also vary upon the drug interaction which may occur. The said daily dosing regimen may also vary upon the additional biological effect(s), other than the prevention of pregnancy, which may be sought through the administration of DRSP.

The daily dosing regimen of DRSP to be daily administered to a female patient may be lower or higher than the doses previously mentioned. For example, a female patient in peri-menopause may require a higher or lower daily dosage of DRSP, in order to improve her general conditions and, for example, in order to improve the regularity of her menstrual cycles.

The adjustment of the daily dosing regimen may be routinely determined by practitioners.

In a preferred embodiment, the pharmaceutical composition further comprises one or more pharmaceutically acceptable excipients.

The pharmaceutical composition may be formulated according to standard methods such as those described in Remington: The Science and Practice of Pharmacy (Lippincott Williams & Wilkins; Twenty first Edition, 2005)

Pharmaceutically acceptable excipients that may be used to formulate the contraceptive composition are, in particular, described in the Handbook of Pharmaceuticals Excipients, American Pharmaceutical Association (Pharmaceutical Press; 6th Revised edition, 2009).

Examples of appropriate excipients include, but are not limited to, fillers, carriers, diluents, binders, anti-caking agents, plasticizers, disintegrants, lubricants, flavors, buffering agents, stabilizers, colorants, dyes, anti-oxidants, anti-adherents, softeners, preservatives and glidants.

In some embodiments, the contraceptive composition comprises one or more excipients selected from the group of binders, fillers, glidants and lubricants.

Examples of fillers include, without being limited to, lactose anhydrous, microcrystalline cellulose, starch, pregelatinized starch, modified starch, dibasic calcium phosphate dihydrate, calcium sulphate trihydrate, calcium sulphate dihydrate, calcium carbonate, lactose, dextrose, sucrose, mannitol and sorbitol and combinations thereof.

Examples of lubricants include, without being limited to, magnesium stearate, calcium stearate, zinc stearate, talc, propylene glycol, PEG, stearic acid, vegetable oil, sodium benzoate, sodium lauryl sulfate, magnesium lauryl sulfate, mineral oil polyoxyethylene monostearate and combinations thereof.

Examples of binders include, without being limited to, starches, e.g., potato starch, wheat starch, corn starch; gums, such as gum tragacanth, acacia gum and gelatin; microcrystalline cellulose, hydroxypropyl cellulose, hydroxyethyl cellulose and hydroxypropylmethyl cellulose; polyvinyl pyrrolidone and combinations thereof.

Examples of glidants include silicon dioxide, magnesium trisilicate, powdered cellulose, starch, talc and tribasic calcium phosphate.

In a preferred embodiment, the pharmaceutical composition does not comprise a significant amount of surfactant agent. A significant amount of a surfactant agent may impair the *in vitro* dissolution profile of DRSP by increasing its initial rate of dissolution. Surfactant agents include non-ionic surfactants such as polyoxyethylene sorbitan fatty acid esters and ionic surfactants such as sodium lauryl sulphate.

It goes without saying that the DRSP to be used may be in a particle form having the specific surface area and/or the d₉₀, d₁₀ and d₅₀ particle sizes which are fully-described in the present specification.

The said contraceptive composition may optionally comprise additional excipients which may accounts for about 0.1 % to 10% by weight.

The contraceptive composition may be formulated in a galenic form suitable for oral administration. Such forms include, without being limited to, tablets, caplets, granules, pills, capsules, powders and suspension.

In preferred embodiments, the contraceptive composition is formulated in a solid form for oral administration such as tablets, capsules, granules, caplets and pills.

Such solid forms are particularly appropriate to be used as daily active dosage unit in the contraceptive kit described herein.

When the pharmaceutical composition is formulated in solid forms such as tablets or pills, the said solid forms may be conveniently coated with a suitable film-forming agent such as hydroxypropylmethyl cellulose, hydroxypropyl cellulose or ethyl cellulose, to which a suitable excipient may optionally be added, e. g. a softener such as glycerol, propylene glycol, diethylphthalate or glycerol triacetate, a filler such as sucrose, sorbitol, xylitol, glucose or lactose, or a colorant such as titanium hydroxide, etc.

The pharmaceutical composition in the form of tablets, pills or granules may be prepared by conventional methods such as direct compression, dry granulation and wet granulation.

In some embodiments, the solid forms are obtained by direct compression.

A further object of the disclosure is to provide a method for preparing the contraceptive composition as described herein which comprises the steps consisting of:
(i) providing DRSP in a particle form as fully-described previously in the present specification
(ii) providing one or more pharmaceutically acceptable excipients; and
(iii) mixing the DRSP provided in step (i) with the one or more excipients provided in step (ii).

As fully-described above, the Applicant provides technical guidelines to obtain a composition comprising DRSP in a form such that:
(i) no more than 50% of the DRSP initially present in the said composition is dissolved within 30 minutes and
(ii) at least 50% of the said DRSP is dissolved in a time range from 3 hours to 4 hours,
when the composition is subjected to an *in vitro* dissolution test, the percentages of DRSP being related to the amount of DRSP initially present in the said composition.

A DRSP containing composition with such an *in vitro* dissolution profile or the *in vivo* pharmacokinetic profile fully-described above may be achieved by various other ways.

By routine experiments and in view of his general knowledge, the one skilled in the art may modify (i) the particle size distribution of DRSP and (ii) the amounts and the nature of excipients in order to obtain other alternative compositions displaying the *in vitro* dissolution profile and the *in vivo* pharmacokinetic profile described in the present application.

For example, the one skilled in the art may conceive a composition comprising (i) micronized DRSP together with (ii) a slow release agent in order to diminish the dissolution rate of said DRSP.

The one skilled in the art may also contemplate to combine (i) large particles of DRSP together with (ii) a surfactant and/or a wetting agent in order to ensure the dissolution of said DRSP.

In certain embodiments, said pharmaceutical composition comprises a contraceptive kit comprising one or more packaging units wherein each packaging unit comprises 21 to 28 daily active dosage units and wherein:
(a) the amount of drospirenone in each daily active dosage unit is at least 3 mg without estrogen, and
(b) each daily active dosage unit comprises drospirenone in a form such that:
   (i) no more than 50% of the drospirenone initially present in the said daily active dosage unit is dissolved within 30 minutes and
   (ii) at least 50% of the said drospirenone is dissolved in a time range from 3 hours to 4 hours,
when the daily active dosage unit is subjected to an *in vitro* dissolution test according to the USP XXIII Paddle Method, the percentages of drospirenone being related to the amount of drospirenone initially present in the said daily active dosage unit.

The said contraceptive kit comprises one or more packaging units.

One or more packaging units includes, without being limited to, 1 packaging unit, 2 packaging units, 3 packaging units, 4 packaging units, 5 packaging units and 6 packaging units.

Each packaging unit comprises from 21 to 28 daily active dosage units. As fully described above, each daily active dosage unit consists of a contraceptive composition.

As fully described above, the daily active dosage units preferably do not comprise any estrogen or estrogen derivative such as ethinyl estradiol, mestranol or 8-prenylnaringenin. In other words, the DRSP is preferably present in the daily active dosage units without estrogen.

In more preferred embodiments, DRSP is the sole contraceptive ingredient comprised within the daily active dosage units.

Each packaging unit optionally comprises from 1 to 7 daily dosage units of a pharmaceutically acceptable placebo.

In some embodiments, the contraceptive kit is characterized in that each packaging unit comprises 28 daily dosage units and no daily dosage unit of a pharmaceutically acceptable placebo. Such a contraceptive kit is particularly appropriate to perform the contraceptive method described herein which consists in administering "continuously" DRSP without free-contraceptive period.

In other embodiments each packaging unit comprises:
- 21 to 27 active daily dosage units consisting of a contraceptive composition as fully described in the present application and
- optionally, 1 to 7 daily dosage units of a pharmaceutically acceptable placebo.

Such a contraceptive kit is particularly appropriate to perform the contraceptive method described herein which comprises:
- a first phase wherein active daily dosage units as described herein which do not comprise estrogen are administered to the female patient affected with obesity over a period of 21 to 27 consecutive days followed by;
- a second phase wherein no contraceptive composition is administered to the female patient over a period of 1 to 7 consecutive days.

In some other embodiments, each packaging unit of the kit comprises 24 daily dosage units comprising an effective amount of a contraceptive composition as described herein and, optionally, 4 daily dosage units of a pharmaceutically acceptable placebo.

The packaging unit as described above may have one of the conventional forms usually used for oral contraceptives.

For example, the packaging unit may be a conventional blister pack comprising the appropriate number of dosage units in a sealed blister pack with a cardboard, paperboard, foil or plastic backing and enclosed in a suitable cover. Each blister container may be conveniently numbered or marked in order to facilitate compliance.

The packaging unit may contain daily dosage units in the order in which they are to be taken, i.e. starting with the first of the at least 21 dosage units that contain the composition of DRSP optionally followed by 7 or less empty blisters or by 7 or less dosage units that comprise a pharmaceutically acceptable placebo.

The kit described herein may comprise other appropriate components such as instructions for use.

The following examples are illustrative and are not intended to limit the scope of the invention as claimed.

### EXAMPLES

### Example 1 - LF111 (DRSP) treatment decreases the number of days with bleeding and/or spotting

### 1/ Objectives

The study CF111/302 below represents a pivotal, multicentre, double-blind, double-dummy, randomised trial on the contraceptive efficacy, tolerability and safety of LF111 (DRSP) over 9 cycles of 28 days of treatment (24 active test product tablets followed by 4 days of placebo tablets).

The first objective is to demonstrate the contraceptive efficacy of LF111 and the second objective is to demonstrate the safety and tolerability of LF111, especially regarding bleeding pattern.

### 2/ Materials and methods

### a) Test product doses and mode of administration

LF111 film-coated tablets (test product; 24 tablets containing 4 mg DRSP followed by 4 placebo tablets; León Farma) was orally administered during this trial. LF111 tablets are of the following formula:

| | **ingredient** | **mg/tablet** |
|---|---|---|
| **Active ingredient** | Drospirenone¹ | 4.00 |
| **Excipient** | Microcrystalline cellulose | 33.02 |
| | Lactose, anhydrous | 17.50 |
| | Silica, colloidal anhydrous | 0.29 |
| | Magnesium stearate | 0.29 |
| | White coating system | 1.65 |
| **Total** | | 56.75 |

| | | |
|---|---|---|
| ¹ crystallized and non-micronized drospirenone, prepared according to the process similar to that described in WO 2006/061309; | | |

### b) Trial design

This prospective, multicentre, randomised, double-blind, double-dummy trial was conducted on 857 women without uncontrolled current diseases, at risk of pregnancy, at the age of 18-45 years, systolic blood pressure < 140 mmHg, diastolic blood pressure < 90 mmHg followed in approximately 73 centres in Austria, Czech Republic, Germany, Hungary, Poland, Romania, Slovakia and Spain.

After providing informed consent at visit 1a (screening; V 1a) and receiving study medication at visit 1b, subjects will attend visits 2 to 4 at day 24±2 of the 1^{st}, 3^{rd}, and 6^{th} cycle, and visit 5 (V5) at day 29+2 of the 9^{th} cycle. The follow-up (visit 6; V6) will take place 7-10 days after last LF1 1 1 intake.

The planned total duration of the trial was set to be 16 months, with a maximum of 6 months for the enrolment process, a maximum of 9 months for the contraceptive treatment per se and 10 days for the follow-up step. The duration of contraceptive treatment for the individual women is 9 x 28 days.

### c) Exclusion criteria

Pregnant subject; Breast-feeding subject; Subject is known to or suspected of not being able to comply with the study protocol and the use of the IMPs (Investigational Medicinal Products); Abnormal finding on pelvic, breast or intravaginal ultrasound examination that precludes participation in the trial; Unexplained amenorrhoea, known polycystic ovary syndrome; Subject having ASC-US or more severe finding on Pap smear; Known contraindication or hypersensitivity to the active ingredient (drospirenone) or excipients of IMPs (cellulose, lactose, silicon dioxide, magnesium stearate, corn starch, polyethylene glycol, polyvinyl pyrrolidone, polyvinyl alcohol, aquarius BT16035 cottage green, talc, titanium dioxide; silica colloidal anhydrous, all-rac-α-tocopherol, lactose, monohydrate, maize starch, povidone, stearic acid, hypromellose, macrogol 400); Significant cardiovascular, hepatic or renal disease, diabetes with vascular involvement, uncontrolled thyroid disorder or current venous thrombosis or embolism; Undiagnosed vaginal bleeding; Known or suspected sex-steroid sensitive malignancies; Presence or history of severe hepatic disease as long as liver function values have not returned to normal; Evidence or history of alcohol, medication or drug abuse (within the last 12 months); Known bleeding disorder or history of unexplained bleeding or bruising within the last 12 months prior to V1a; Prohibited previous medication / contraceptives (injectable hormonal methods of contraception within the last 6 months before V1a, progestin-releasing IUD (intra uterine device) or contraceptive implant within the last 2 months before V1a, anti-retroviral therapy within the last 6 months before V1a, microsomal enzyme-inducing drugs within the last 28 days before the start of IMP intake); Dependence on prohibited co-medication (estrogens, progestogens, activated charcoal, microsomal enzyme-inducing drugs, anticonvulsants [e.g. hydantoins, phenytoin, carbamazepine, oxcarbazepine, topiramate, felbarnate, primidone], barbiturates, antibiotics [such as rifabutin or rifampicin], ritonavir, nelfinavir, atorvastatin, bosentan, griseoulvin, phenylbutazon, St. John's wort [hypericum perforatum], medications that may increase serum potassium [ACE inhibitors, angiotensin - H receptor antagonists, potassium-sparing diuretics, potassium supplementation, heparin, aldosterone antagonists and NSAIDs]); Planned surgery during the anticipated time of participation in this trial requiring withdrawal of an oral contraceptive; Regular concomitant use of barrier contraceptive methods, spermicides, IUDs or other contraceptive measures (excepting occasional use due to risk of infection); Evidence or history of neurotic personality, psychiatric illness or suicide; Participation in another trial of investigational drugs or devices parallel to, or less than 90 days before trial entry, or previous participation in this trial; Employee of the investigator or trial centre, or family member of the employees or the investigator; Any condition that, in the opinion of the investigator, may jeopardise the trial conduct according to the protocol.

### d) Criteria for evaluation

### d.1) Efficacy

- primary: Overall Pearl Index (overall PI);
- secondary: PI for method failures; PI after correction for back-up contraception; Pregnancy ratio.

### d.2) Safety/Tolerability

Proportion of subjects with unscheduled bleeding/spotting in cycles 2 to 6; Proportion of subjects with unscheduled bleeding/spotting in each cycle from cycles 2 to 9 and cumulative in cycles 2 to 6 and cycles 2 to 9; Number of days of bleeding/spotting during cycles 2 to 4; Number of days of bleeding/spotting during cycles 7 to 9; Number of days of bleeding/spotting during cycles 2 to 9; Number of bleeding/spotting episodes during cycles 2 to 4; Number of bleeding/spotting episodes during cycles 7 to 9; Number of bleeding/spotting episodes during cycles 2 to 9; Proportion of subjects with amenorrhoea; Change in body weight from baseline (V1a); Change in systolic and diastolic blood pressure from baseline (V1a); Adverse events (AEs); Pulse rate; Electrocardiogram (ECG) for a subset of subjects; Clinical laboratory parameters; Special clinical laboratory parameters (haemostatic variables, carbohydrate metabolism and bone metabolism) for a subset of subjects.

### d.3) Statistical methods

### d.3.1) Efficacy parameters:

Analysis of the primary efficacy variable defined as overall PI will be performed for the Full Analysis Set (FAS) and the Per Protocol Set (PPS). Primary assessment of efficacy will be based on the FAS. Two-sided 95% confidence interval (CI) for overall PI will be calculated assuming that events of pregnancy have a Poisson distribution. Secondary efficacy analysis will be based on the FAS. Two-sided 95% Cls will be calculated for the method failures PI. The Clopper-Pearson 95% confidence interval will be calculated for the pregnancy ratio. The cumulative pregnancy rate will be calculated using the Kaplan Meier estimator. Two sided 95% CI will be calculated for the PI after correction for back-up contraception.

### d.3.2) Safety and tolerability parameters

Analysis of the parameters blood pressure, body weight, and bleeding pattern will be based on FAS.. Analysis of safety endpoints will be conducted using the Safety Set only. All adverse events (AEs) and treatment-emergent adverse events (TEAEs) will be summarised by calculating the number and percent of subjects with AEs by preferred term and system organ class. Also TEAEs will be summarised by severity and relationship to treatment. Number and percent of TEAEs leading to study termination will be provided. Laboratory parameters, pulse rate and abnormal ECG results (e.g. QT prolongation) will be summarised by calculating summary statistics on the absolute values and on the change from V1a (special laboratory parameters and ECG: V1b) to V3, V4 and V5. Shift tables will be provided to illustrate changes with respect to the laboratory normal ranges between V1a and V5 (or EDV). The number and percent of subjects with values outside the limits of clinical significance will be summarised.

### 2/ Results

As shown in Table 1 below, there is a significant reduction in the number of days with bleeding and/or spotting for the DRSP treatment in women with excess weight (BMI of 25 kg/m² or greater) as compared with non-overweight women subjected to the same contraceptive treatment.

**Table 1: Number of days with spotting and/or bleeding by period of treatment cycles for a cohort of women with excess weight, as compared with a cohort of women with no excess weight for the DRSP treatment.**

| **Cycle** | | **BMI<25 kg/m² (N=660)** | **% per treatment cycle** | **BMI≥25 kg/m² (N=198)** | **% per treatment cycle** | **Total (N=858)** | **Wilcoxon-rank-sum-test p value** |
|---|---|---|---|---|---|---|---|
| **Cycles 2 - 4** | n | 401 | | 126 | | 527 | |
| | Mean (SD) | 13.8 (12.84) | 16.4% | 10.7 (13.46) | 12.7% | 13.1 (13.05) | 0.0007 |
| | Median | 11.0 | | 6.0 | | 10.0 | |
| | Min/ Max | 0/60 | | 0/66 | | 0/66 | |
| | | | | | | | |
| **Cycles 2 - 6** | n | 315 | | 107 | | 422 | |
| | Mean (SD) | 20.6 (18.62) | 14.7% | 14.9 (18.64) | 10.6% | 19.1 (18.77) | 0.0005 |
| | Median | 17.0 | | 7.0 | | 14.0 | |
| | Min/ Max | 0/100 | | 0/89 | | 0/100 | |
| | | | | | | | |
| **Cycles 2 - 9** | n | 221 | | 84 | | 305 | |
| | Mean (SD) | 32.1 (27.85) | 14.3% | 22.2 (26.65) | 9.9% | 29.4 (27.84) | 0.0010 |
| | Median | 26.0 | | 13.5 | | 21.0 | |
| | Min/ Max | 0/109 | | 0/106 | | 0/109 | |
| | | | | | | | |
| **Cycles 5 - 7** | n | 315 | | 108 | | 423 | |
| | Mean (SD) | 10.8 (11.13) | 12.8% | 8.3 (10.98) | 9.9% | 10.2 (11.13) | 0.0053 |
| | Median | 7.0 | | 4.0 | | 6.0 | |
| | Min/ Max | 0/67 | | 0/49 | | 0/67 | |
| | | | | | | | |
| **Cycles 7 - 9** | n | 280 | | 94 | | 374 | |
| | Mean (SD) | 10.2 (10.10) | 12.1% | 7.9 (11.09) | 9.4% | 9.7 (10.39) | 0.0040 |
| | Median | 8.0 | | 3.5 | | 6.0 | |
| | Min/ Max | 0/60 | | 0/55 | | 0/60 | |
| | | | | | | | |

| | | | | | | | |
|---|---|---|---|---|---|---|---|
| N: Number of subjects in the Test group in the particular BMI group n: Number of subjects with data available SD: Standard deviation | | | | | | | |

As shown in Table 2 below, there is a significant reduction in the number of days with bleeding and/or spotting for the DRSP treatment in obese women as compared with non-obese women subjected to the same contraceptive treatment.

**Table 2: Number of days with spotting and/or bleeding by period of treatment cycles for a cohort of obese women, as compared with a cohort of non-obese women for the DRSP treatment.**

| **Cycle** | | **BMI<30 kg/m² (N=828)** | **% per treatment cycle** | **BMI≥30 kg/m² (N=30)** | **% per treatment cycle** | **Total (N=858)** | **Wilcoxon-rank-sum-test p value** |
|---|---|---|---|---|---|---|---|
| Cycles 2 - 4 | n | 511 | | 16 | | 527 | |
| | Mean (SD) | 13.3 (13.13) | 15.8% | 5.3 (6.66) | 6.3% | 13.1 (13.05) | 0.0097 |
| | Median | 10.0 | | 2.0 | | 10.0 | |
| | Min/ Max | 0/66 | | 0/22 | | 0/66 | |
| | | | | | | | |
| Cycles 2 - 6 | n | 408 | | 14 | | 422 | |
| | Mean (SD) | 19.6 (18.88) | 14.0% | 6.3 (7.89) | 4.5% | 19.1 (18.77) | 0.0053 |
| | Median | 16.0 | | 2.0 | | 14.0 | |
| | Min/ Max | 0/100 | | 0/24 | | 0/100 | |
| | | | | | | | |
| Cycles 2 - 9 | n | 291 | | 14 | | 305 | |
| | Mean (SD) | 30.4 (28.02) | 13.6% | 9.2 (12.10) | 4.1% | 29.4 (27.84) | 0.0027 |
| | Median | 22.0 | | 2.0 | | 21.0 | |
| | Min/ Max | 0/109 | | 0/36 | | 0/109 | |
| | | | | | | | |
| Cycles 5 - 7 | n | 405 | | 18 | | 423 | |
| | Mean (SD) | 10.4 (11.18) | 12.4% | 5.4 (8.76) | 6.4% | 10.2 (11.13) | 0.0217 |
| | Median | 7.0 | | 1.5 | | 6.0 | |
| | Min/ Max | 0/67 | | 0/29 | | 0/67 | |
| | | | | | | | |
| Cycles 7 - 9 | n | 356 | | 18 | | 374 | |
| | Mean (SD) | 10.0 (10.47) | 11.9% | 3.8 (6.62) | 4.5% | 9.7 (10.39) | 0.0037 |
| | Median | 7.0 | | 0.0 | | 6.0 | |
| | Min/ Max | 0/60 | | 0/20 | | 0/60 | |
| | | | | | | | |

| | | | | | | | |
|---|---|---|---|---|---|---|---|
| N: Number of subjects in the Test group in the particular BMI group n: Number of subjects with data available SD: Standard deviation | | | | | | | |

### Example 2 - Correlation between BMI and DRSP-based treatment on one hand and change in weight and change in heart rate on the other hand

### 1/ Methods

In example 2, the LF111 formulation described in Example 1 was used.

The CF111/301 clinical trial protocol included 713 healthy sexually active women willing to use oral contraceptives enrolled in approximately 41 centres located in 5 countries (Hungary, Poland, the Czech Republic, Germany and Romania).

After signing an Informed Consent Form at visit 1a (screening) and receiving study medication at visit 1b, eligible subjects attended visits 2 to 6 at day 24±2 of the 1^{st}, 3^{rd}, 6^{th}, 9^{th} and 13^{th} cycle. The follow-up (visit 7) took place 10 to 28 days after visit 6. At least 515 subjects completed the study with 13 cycles each.

Data set had information on demographic and clinical parameters, gynaecological and medical history data, laboratory and vital signs assessments, prior/concomitant medications/contraceptive devices related data.

Statistical p values were calculated by using a Fisher exact test, and p value was found significant test at the threshold p≤0.05.

### 2/ Results

For women affected with obesity (BMI ≥30 kg/m²) a trend in decreasing their weight and heart rate from visit 1 (measured at baseline) to visit 6 (measured at the end of the study) was observed.

Distribution of the change in weight and heart rate from visit 1 to 6 for women by BMI group (BMI < 30 and BMI ≥ 30 kg/m²) is shown in Figures 1 and 2 respectively.

The linear model analysis showed that the effect of BMI group was statistically significant in the change of weight (F-statistic: 14.49 on 1 and 668 DF, p-value: 0.0001541) and heart rate (F-statistic: 4.947 on 1 and 666 DF, p-value: 0.02647) from visit 1 to 6.

### Example 3 - Reduced adverse effects of the DRSP-based treatment in obese women

### 1/ Methods

The CF111/1SS clinical trial protocol included 1571 (1500 + 71 ) healthy sexually active women willing to use oral contraceptives enrolled in approximately 114 centres located in in Austria, Czech Republic, Germany, Hungary, Poland, Romania, Slovakia and Spain. A group of non-obese women (BMI< 30 kg/m²) and of obese women (BMI≥ 30 kg/m²) were studied separately.

### 2/ Results

Table 3 depicts the results of quantification of TEAEs (Treatment Emergent Adverse Events) in a cohort of women subject to contraception with a drospirenone-containing composition ("LF111"). Non-obese women (BMI< 30 kg/ m²) and obese women (BMI≥ 30 kg/m²) were studied, respectively.

The results disclosed in Table 3 show that the percentage of TEAEs was similar in women subjected to the DRSP-POC formulation, irrespective of whether these women are obese women or non-obese women.

Thus, the results show that the drospirenone-containing formulation shall be endowed with a high observance rate by obese women.

**Table 3: Incidence of TEAEs by BMI subgroup of individuals treated with LF111**

| | BMI<30 kg/m² (N=1500) | BMI≥30 kg/m² (N=71) |
|---|---|---|
| | n (%) | n (%) |
| Subjects with at least one TEAEs [a] | 650 (43.3) | 30 (42.3) |

| | | |
|---|---|---|
| N: Number of subjects in specified treatment group. n: Number of subjects with adverse events. %: Percentage based on N. [a] TEAE: Treatment Emergent Adverse Event. TEAEs are defined as AEs which started at or after the first administration of an IMP and includes those events started prior to the first administration of an IMP but which worsened after the first intake. Adverse events starting after the last administration of an IMP but within the follow up period after last IMP will be regarded as treatment-emergent. | | |

Heart rate is understood as the number of times a person's heart beats per minute at rest (e.g., not exercising). Preferably, heart rate may be measured after a patient has been lying down for at least 5 minutes, preferably for at least 10 minutes, and most preferably for at least 15 minutes. Alternatively, heart rate may be measured upon waking in the morning and before rising from bed. Heart rate is an important indicator of health.

While a normal heart rate for adults may range from about 60 to about 100 beats per minute, a lower heart rate is indicative of a more efficient heart function and of cardiovascular fitness. While overweight and obese women are generally observed to have a higher heart rate than women of normal weight, it has also been found that a faster heart rate is a warning sign for increased cardiovascular problems and also as a predictor for obesity later in life. Women with higher heart rates have been found to be predisposed to obesity and diabetes mellitus. Shigetoh, et al., Am. J. Hypertension, vol. 22, number 2, pp. 151-155, Feb. 2009. Higher heart rates are believed to be associated with metabolic syndrome, diabetes, formation of blood clots that can cause a stroke or heart attack, heart failure, fainting spells, and even sudden death.

Thus, the reduction in heart rate is extremely desirable, especially for overweight or obese women, as lowering heart rate may result in reducing the risk of developing various deleterious health conditions. It is believed that reductions in heart rate of at least 5 beats per minute, of at least 10 beats per minute and at least 15 beats per minute will provide significant reductions of such risk factors.

### Example 4 - Comparison of the bleeding or spotting events observed in obese women, with a DRSP-based treatment and a treatment based on desogestrel

### 1/ Objectives

The study CF111/302 below represents a pivotal, multicentre, double-blind, double-dummy, randomised trial on the contraceptive efficacy, tolerability and safety of LF111 (DRSP) over 9 cycles in comparison with desogestrel 0.075 mg (Cerazette).

The first objective is to demonstrate the contraceptive efficacy of LF111 and the second objective is to demonstrate the safety and tolerability of LF111 in comparison to desogestrel 0.075 mg, especially regarding bleeding pattern.

### 2/ Materials and methods

### a) Test and reference products, doses and mode of administration

Two kinds of tablets were orally administered during this trial:
- LF111 film-coated tablets (test product; 24 tablets containing 4 mg DRSP followed by 4 placebo tablets; León Farma) was orally administered during this trial.
The formula of LF111 tablets is disclosed in example 1 (see section 2, subsection a)).
- Desogestrel 0.075 mg film-coated tablets (reference product; 28 active tablets; NV Organon).

### b) Trial design

This prospective, multicentre, randomised, double-blind, double-dummy trial was conducted on 1200 women without uncontrolled current diseases, at risk of pregnancy, at the age of 18-45 years, systolic blood pressure < 140 mmHg, diastolic blood pressure < 90 mmHg (857 for LF111 and 343 for desogestrel 0.075 mg; Randomisation ratio 5:2) followed in approximately 88 centres in Austria, Czech Republic, Germany, Hungary, Poland, Romania, Slovakia and Spain.

After providing informed consent at visit 1a (screening; V 1a) and receiving study medication at visit 1b, subjects will attend visits 2 to 4 at day 24±2 of the 1^{st}, 3^{rd}, and 6^{th} cycle, and visit 5 (V5) at day 29+2 of the 9^{th} cycle. The follow-up (visit 6; V6) will take place 7-10 days after last LF1 1 1 intake.

The planned total duration of the trial was set to be 16 months, with a maximum of 6 months for the enrolment process, a maximum of 9 months for the contraceptive treatment per se and 10 days for the follow-up step. The duration of contraceptive treatment for the individual women is 9 x 28 days.

### c) Exclusion criteria

See the corresponding section of example 1 above.

### d) Criteria for evaluation

See the corresponding section of example 1 above.

### 3/ Results

The Tables 4 and 5 show the comparison of the bleeding or spotting events observed in obese women, with a DRSP-based treatment (LF111) and a treatment based on desogestrel (commercially available as Cerazette^{®} pills).

**Table 4: Bleeding or spotting events observed in obese female individuals (BMI ≥ 30 kg/m²), with respect to the treatment undertaken**

| **Bleeding or spotting Mean # days** | **BMI ≥ 30 kg/m²** | |
|---|---|---|
| | **DRSP-based treatment** | **desogestrel** |
| Period cycles 2-4 | 5.3 | 21.8 |
| Period cycles 2-6 | 6.3 | 16.3 |
| Period cycles 2-9 | 9.2 | 33.9 |

**Table 5: Bleeding or spotting events observed in normal or over-weight female individuals (BMI < 30 kg/m²), with respect to the treatment undertaken**

| **Bleeding or spotting Mean # days** | **BMI < 30 kg/m²** | |
|---|---|---|
| | **DRSP-based treatment** | **desogestrel** |
| Period cycles 2-4 | 13.3 | 16.6 |
| Period cycles 2-6 | 19.6 | 24.1 |
| Period cycles 2-9 | 30.4 | 34.7 |

It is observed that obese female individuals that are administered with the drospirenone-based contraceptive treatment, as disclosed herein, suffer significantly less bleeding or spot events during the all the periods cycles that were analysed, as compared to obese female individuals undertaking a treatment based on a desogestrel-containing contraceptive composition.

Contrarily, female individuals having a BMI lower than 30 kg/m², including individuals with a normal weight or individuals with excess weight, suffer from identical or equivalent of bleeding or spotting events, irrespective of the contraceptive treatment that is undertaken.

## Claims

1. Drospirenone as the sole contraceptive ingredient comprised in a daily active dosage unit in an amount of at least 3 mg for use in therapy as a contraceptive in a female patient affected with obesity, as featured by a BMI of 30 kg/m² or greater.

2. Drospirenone for use according to claim 1, wherein said use in therapy is the reduction in the number of days with bleeding events.

3. Drospirenone for use according to any of claims 1 or 2, wherein the said daily active dosage unit is comprised within a contraceptive kit comprising one or more packaging units wherein each packaging unit comprises 21 to 28 daily active dosage units and wherein:
a. the amount of drospirenone in each daily active dosage unit is at least 3 mg without estrogen, and
b. each daily active dosage unit comprises drospirenone in a form such that:
i. no more than 50% of the drospirenone initially present in the said daily active dosage unit is dissolved within 30 minutes and
ii. at least 50% of the said drospirenone is dissolved in a time range from 3 hours to 4 hours,
when the daily active dosage unit is subjected to an *in vitro* dissolution test according to the USP XXIII Paddle Method, the percentages of drospirenone being related to the amount of drospirenone initially present in the said daily active dosage unit.

4. Drospirenone for use according to any of claims 1 to 3, wherein the amount of drospirenone in each daily active unit dosage ranges from about 3.5 mg to 4.5 mg.

5. Drospirenone for use according to any one of claims 2 or 3, wherein the one or more packaging units further comprise from 1 to 7 daily dosage units of a pharmaceutically acceptable placebo.

6. Drospirenone for use according to any of claims 3 to 5, wherein each packaging unit comprises 24 daily active dosage units.

7. Drospirenone for use according to any of claims 3 to 6, wherein each packaging unit comprises 4 daily placebo dosage units.

8. Drospirenone for use according to any of claims 1 to 7, wherein the said active ingredient is in a crystalline form.

9. Drospirenone for use according to any of claims 1 to 8, wherein the said active ingredient is in a non-micronized form.

10. Drospirenone for use according to any of claims 1 to 9, wherein the said active ingredient has a d₅₀ of less than 70 µm

11. Drospirenone for use according to any one of claims 1 to 10, wherein the said active ingredient in a particle form has a specific surface area from about 2,000 cm²/g to about 8,500 cm²/g.

## Patentansprüche

1. Drospirenon als einziger empfängnisverhütender Inhaltsstoff umfasst in einer täglichen aktiven Dosierungseinheit in einer Menge von mindestens 3 mg für dessen Verwendung in der Therapie als Verhütungsmittel in einem weiblichen, von Fettleibigkeit betroffenen Patienten, wie durch einen BMI von 30 kg/m² oder größer charakterisiert.

2. Drospirenon für dessen Verwendung nach Anspruch 1, wobei die genannte Verwendung in der Therapie die Verringerung der Anzahl von Tagen mit Blutungsereignissen ist.

3. Drospirenon für dessen Verwendung nach einem der Ansprüche 1 oder 2, wobei die genannte tägliche aktive Dosierungseinheit innerhalb eines empfängnisverhütenden Kits umfasst ist, umfassend eine oder mehrere Verpackungseinheiten, wobei jede Verpackungseinheit 21 bis 28 tägliche aktive Dosierungseinheiten umfasst, und wobei:
a. die Menge von Drospirenon in jeder täglichen aktiven Dosierungseinheit mindestens 3 mg ohne Östrogen ist, und
b. jede tägliche aktive Dosierungseinheit Drospirenon in einer Form umfasst, sodass:
i. nicht mehr als 50 % des anfänglich in der genannten täglichen aktiven Dosierungseinheit vorhandenen Drospirenons innerhalb von 30 Minuten gelöst wird, und
ii. mindestens 50 % des genannten Drospirenons in einer Zeitspanne von 3 Stunden bis 4 Stunden gelöst wird,
wenn die tägliche aktive Dosierungseinheit einem *In vitro*-Auflösungstests gemäß der USP XXIII Paddle-Methode unterzogen wird, wobei sich die Anteile von Drospirenon auf die Menge von anfänglich in der genannten täglichen aktiven Dosierungseinheit vorhandenem Drospirenon beziehen.

4. Drospirenon für dessen Verwendung nach einem der Ansprüche 1 bis 3, wobei die Menge von Drospirenon in jeder täglichen aktiven Dosierungseinheit zwischen etwa 3,5 mg und 4,5 mg liegt.

5. Drospirenon für dessen Verwendung nach einem der Ansprüche 2 oder 3, wobei die eine oder mehreren Verpackungseinheiten zusätzlich von 1 bis 7 täglichen Dosierungseinheiten einer pharmazeutisch akzeptablen Placebos umfassen.

6. Drospirenon für dessen Verwendung nach einem der Ansprüche 3 bis 5, wobei jede Verpackungseinheit 24 tägliche aktive Dosierungseinheiten umfasst.

7. Drospirenon für dessen Verwendung nach einem der Ansprüche 3 bis 6, wobei jede Verpackungseinheit 4 tägliche Placebo-Dosierungseinheiten umfasst.

8. Drospirenon für dessen Verwendung nach einem der Ansprüche 1 bis 7, wobei der genannte aktive Inhaltsstoff in einer kristallinen Form vorliegt.

9. Drospirenon für dessen Verwendung nach einem der Ansprüche 1 bis 8, wobei der genannte aktive Inhaltsstoff in einer nicht-mikronisierten Form vorliegt.

10. Drospirenon für dessen Verwendung nach einem der Ansprüche 1 bis 9, wobei der genannte aktive Inhaltsstoff einen d₅₀ von weniger als 70 µm aufweist.

11. Drospirenon für dessen Verwendung nach einem der Ansprüche 1 bis 10, wobei der genannte aktive Inhaltsstoff in Partikelform eine spezifische Oberfläche von etwa 2.000 cm²/g bis etwa 8500 cm²/g aufweist.

## Revendications

1. Drospirénone en tant que seul principe contraceptif compris dans une unité de dosage actif quotidien dans une quantité d'au moins 3 mg pour son utilisation en thérapie comme contraceptif chez un patient féminin atteint d'obésité, tel que mesuré par un IMC égal à 30 kg/m² ou supérieur.

2. Drospirénone pour son utilisation selon la revendication 1, dans laquelle ladite utilisation en thérapie est la réduction du nombre de jours avec des événements de saignement.

3. Drospirénone pour son utilisation selon l'une quelconque des revendications 1 ou 2, dans laquelle ladite unité de dosage actif quotidien est comprise à l'intérieur d'un kit contraceptif comprenant une ou plusieurs unités d'emballage dans laquelle chaque unité d'emballage comprend 21 à 28 unités de dosage actif quotidien et dans laquelle :
a. la quantité de drospirénone dans chaque unité de dosage actif quotidien est au moins de 3 mg sans oestrogène, et
b. chaque unité de dosage actif quotidien comprend de la drospirénone sous une forme telle que :
i. pas plus de 50 % de la drospirénone initialement présente dans ladite unité de dosage actif quotidien est dissoute dans un délai de 30 minutes et
ii. au moins 50 % de ladite drospirénone est dissoute dans un intervalle de temps allant de 3 heures jusqu'à 4 heures,
lorsque l'unité de dosage actif quotidien est soumise à un test de dissolution *in vitro* selon la méthode à pales USP XXIII, les pourcentages de drospirénone étant en rapport avec la quantité de drospirénone initialement présente dans ladite unité de dosage actif quotidien.

4. Drospirénone pour son utilisation selon l'une quelconque des revendications 1 à 3, dans laquelle la quantité de drospirénone dans chaque unité de dosage actif quotidien varie d'environ 3,5 mg à 4,5 mg.

5. Drospirénone pour son utilisation selon l'une quelconque des revendications 2 ou 3, dans laquelle l'une ou plusieurs unités d'emballage comprennent en outre de 1 à 7 unités de dosage quotidien d'un placebo pharmaceutiquement acceptable

6. Drospirénone pour son utilisation selon l'une quelconque des revendications 3 à 5, dans laquelle chaque unité d'emballage comprend 24 unités de dosage actif quotidien.

7. Drospirénone pour son utilisation selon l'une quelconque des revendications 3 à 6, dans laquelle chaque unité d'emballage comprend 4 unités de dosage de placebo quotidien.

8. Drospirénone pour son utilisation selon l'une quelconque des revendications 1 à 7, dans laquelle ledit principe actif est sous une forme cristalline.

9. Drospirénone pour son utilisation selon l'une quelconque des revendications 1 à 8, dans laquelle ledit principe actif est sous une forme non micronisée.

10. Drospirénone pour son utilisation selon l'une quelconque des revendications 1 à 9, dans laquelle ledit principe actif a un d₅₀ de moins de 70 µm.

11. Drospirénone pour son utilisation selon l'une quelconque des revendications 1 à 10, dans laquelle ledit principe actif sous forme d'une particule a une surface spécifique d'environ 2 000 cm²/g à environ 8 500 cm²/g.
